# EUROPEAN PATENT SPECIFICATION

(11) **EP 1 781 110 B1**
(45) Date of publication and mention of the grant of the patent: **17.02.2010**
(21) Application number: 04738998.6
(22) Date of filing: 09.07.2004
(51) Int. Cl.: A22C 17/10, A22B 5/00, A22C 18/00, G01N 33/12

(54) **QUALITY CONTROL SYSTEM**
QUALITÄTSSICHERUNGSSYSTEM
SYSTEME DE CONTROLE DE LA QUALITE

(43) Date of publication of application: 09.05.2007
(73) Proprietor: CARNITECH A/S, 9530 Stovring (DK)
(72) Inventor: CARLSEN, Flemming, DK-9440 Abybro (DK)
(74) Representative: Tellefsen, Jens J.
(86) International application number: PCT/DK2004/000501
(87) International publication number: WO 2006/005335

(56) References cited:
- EP-A- 0 806 683
- EP-A- 0 899 589
- EP-A2- 1 716 755
- US-A- 5 937 080
- US-A- 6 104 966
- US-A1- 2002 004 366
- US-B1- 6 649 412

## Description

### Field of invention

The present invention relates to a quality control system for continuous control of meat products. Such systems will find widespread use, especially in meat processing plants, slaughterhouses, abattoirs and the like Examples of these systems are disclosed in documents US 5 937 080 and US 2002/004 366.

### Background of the invention

There is a general trend in society, in particular in the Western world, that food stuffs which are being treated industrially shall be monitored such that it will be possible to trace the finished product all the way back to the farm and the farmer which originally grew or produced the food stuff. Additionally, there is an increasing desire to know that the food stuff bought by the consumer has been treated in a hygienic, safe and controlled manner such that risks from contamination, inclusion of foreign objects, bacteria growth and the like has been avoided or at least been minimized, and/or that the source of such contamination may easily and quickly be traced in order to recall food stuffs which do not fulfil the requirements set by society.

Another aspect of producing food stuffs is to be able to certify that the product which the consumer buys, actually is the product which is claimed to be contained in the package.

When processing meat, and especially when producing mince meats, the finished product is often labelled according to date of production, i.e. the date on which the meat was minced, packed in the tray, weight of the meat portion, and also often an interval relating to the fat content is mentioned, as for example "8-10% fat". Industrialized meat production is often carried out such that relatively large batches of meat is minced and transferred to one large container.

By laboratory test or other methods for deciding the fat content, the average fat content of the container is determined. When it is desirable to mix a minced meat portion having a certain fat content, minced meat from different containers may be combined in order to reach the desired fat content or at least a fat content within a certain interval, for example 8-10%. Often this mixing of minced meat from different batches is done according to experience. This, in turn, generates a number of possibilities for error. Firstly, the minced meat in one batch may have been determined by laboratory tests to have a certain fat content. The batch is typically 500-1500 kg, whereas a test sample which is used to determine the fat content may only be a few grams, such that the test sample may not be indicative of the entire batch. By then combining two batches or more, where the actual fat content might be different than the fat content determined by laboratory tests, the accumulated error might be so large that even though an interval of 2%, for example 8-10%, is indicated on the label of the package, the fat content may be smaller or larger. The consumer thereby is misinformed about the content of the package.

Commercial systems are available which will determine the fat content of meat, either as a whole piece of meat or as shredded/minced meat. These methods include X-ray devices as suggested by Smiths and Heimann, Foss and others (GB 1077868), or by radio waves as suggested by Carlsberg (US 4631413). All these systems have some drawbacks, but common for the systems using X-rays or radio waves is the fact that the devices must comprise special means for avoiding radiation spreading to the working environment in which the installation is placed. Especially for X-ray devices, the health risks associated with these rays have proven to be larger than what was thought previously. Furthermore, X-ray radiation is considered a cause of some types of cancer. Therefore, devices of this type where X-ray devices are incorporated usually contain some kind of lead curtains or lead covered hatches such that the spreading of radiation is minimized. It should, however, be noticed that this causes quite cumbersome, heavy and often complicated devices, which from a service point of view, and thereby especially for cleaning purposes, do not always provide optimal conditions.

In US 5.937.080 a computer controlled system and method are disclosed, by which system it is possible to among other characteristics determine the fat content and the location of the fat on a piece of meat. This information is used for further processing of the meat in order to trim the meat according to predetermined specifications.

The system and method as such do not certify or in any other way provide information to the end user, i.e. the consumer, about the product, but provide data input for the further processing of the meat products.

It is generally known in the field that devices for producing fresh food stuffs must be engineered such that, firstly, the risk of fresh food stuffs becoming stuck in the machine itself is minimized and, on the other hand, the design of the machine should facilitate effective cleaning in that extremely high standards of hygiene usually are associated with producing fresh food stuff.

### Summary of the invention

It is, therefore, the object of the present invention to provide a quality control system which actually carries out an in situ test and collection of data in a continuous manner from the finished food products and, in particular, fresh minced meat portions. The present invention addresses this by providing a quality control system according to claim 1 for continuous control of meat products being particular in that the system comprises a conveyor onto which the meat products are placed and conveyed, where non-contact means for registering the fat and/or protein and/or moisture content of the meat products is provided for continuous registration as the meat products passes the means on the conveyor, and further a check weighing system comprising a detection unit for detecting foreign objects arranged in connection with the conveyor, and that input from the non-contact fat and/or protein and/or moisture registering means, the weighing system and the metal detection unit are collected in a data storage means.

By this control system, the meat products in the portion size actually being shipped to the consumer is being controlled, i.e. investigated, such that the actual fat and/or protein and/or moisture content, actual weight of the meat portion, and final quality check regarding foreign objects which might have become lodged in the meat item, is checked such that the possibility of actually providing specific data relating to that particular meat product is possible. Furthermore, by using non-contact means, the risk of further contaminating or any other way polluting the meat products is minimized.

Also, the risk of influencing the measuring means is minimized in that by non-contact means, and building the check weighing system into, or immediately adjacent, the conveyor belt, the meat products do not come into contact with the control system as such, but only the characteristics of that particular meat product is collected and transmitted to a storage means for further treatment. The information collected may also be used as feed-back to the system, such that for example if a weight is registered which is below or above the limit, a corrective signal is transmitted to the unit which determines the actual portion size, and thereby the weight of individual portions such that meat portions of correct weight may be produced.

In a further advantageous embodiment of the invention the meat products are minced meat in pre-specified weight portions and that the meat portions are packaged in trays.

Particularly concerning minced meats, it is very important to convey the correct information to the consumer. Minced meat produced on an industrial scale is usually produced in large batches, where different characteristics of each batch are determined by laboratory tests. During the laboratory tests, relatively small samples of each batch are analysed and it is assumed that the results from the laboratory tests are indicative of the entire batch. When it comes to actually producing the minced meat which is to be packaged and sold to the consumer, the different batches are mixed such that the finished minced meat products will obtain the characteristics desired by the consumer. This may, for example, be the desired fat content, mix of different types of meat, etc.

The assumption that the relatively small sample is representative of the entire batch, which may be 500-1500 kg, often leads to finished mixes of minced meat products which do not fulfil the requirements that were set originally for the mixture of that particular consumer production. It is, therefore, important to be able to verify, after the mixing and packaging has been completed, that the minced meat product does fulfil the required specifications, or, which is possible with the present invention, to indicate on the packaged meat product the exact content relating to fat, liquid, meat, date of production, etc. In this manner, the consumer is provided with specific quality statements relating to that particular minced meat portion.

In a still further advantageous embodiment of the invention, the non-contact means is a device comprising a source of light, for example halogen light, and at least one sensor for receiving/monitoring and transmitting the reflected wavelengths from the products treated with the light to a computing unit, and further that the light device may comprise a non-contact temperature registration means. In relation to other non-contact means, the risk of radiation, or otherwise undesirable influences both on the products to be controlled and on the working environment adjacent the control station, light is not harmful. In systems where X-rays are used, the radiation both from the source of X-ray and from the stray radiation will, over time, increase the risk of being harmful to personnel working in or around such machinery. It has been proven that X-ray radiation is a cause of cancer and it is consequently desirable to avoid being exposed to such radiation. Other sources of radiation, such as radioactive sources, have also been investigated, but again the problems relating to radiation makes these systems undesirable for industrial purposes, and especially for purposes where personal has to work in close proximity to the machinery and where food items are concerned. It is, therefore, advantageous to use a light device where the physical structure of the meat products passing under the light will absorb and reflect the light at different wavelengths whereby it becomes possible, through suitable software, to analyse the absorption/reflection and thereby determine the texture of the material which the light is exposing, and in this manner determine the relationship between meat, fat, proteins, liquid and other properties.

Furthermore, it has been shown that it is possible to determine differences in reflection/absorption, depending on the type of meat being passed under the non-contact light device such that, for industrial purposes it is possible by implementing the software such that different wavelengths corresponds to different meat types, it may be determined which mix of minced meat is being passed through the quality control system.

An important factor when treating fresh food products, and especially meat products, is the fact that the temperature throughout the process should be kept between certain limited intervals, and optimum treatment temperature is from -10° C to 7° C in order to minimize the risk of contamination in the meat products due to bacteria growth etc. It is therefore foreseen that, in addition to the temperature registration during handling, transport, storage and production, the quality control system may also comprise a non-contact temperature registration means in order to verify that also during the last stage of the production process the temperature was within the pre-described interval.

In a further advantageous embodiment the check weighing system is integral with the conveyor belt, and that the check weighing is in communication with a computing unit. By integrating more features in the quality control system in the same units, it becomes possible to create a more compact quality control system. In systems where for example X-ray scanners are used in order to determine the constitution of the meat products, a rather large tunnel around the meat products needs to be provided in order to safeguard against stray radiation from the X-raying source, and furthermore, the X-ray scanning chamber as such needs to have a certain size in order to be able to accommodate the necessary features of such an apparatus inside the tunnel. On the other hand, by using the light, the tunnel may be avoided altogether, which also provides casier access for hygienic control and cleaning purposes, and furthermore, by integrating the check weighing system into the conveyor belt this additional feature may be provided in the same space as the conveying belt.

Also, the collection of data, in addition to providing traceability to the products produced at the single steps, furthermore provides the possibility of feed-back into the process such that small corrections may be carried out continuously in order to assure the best possible quality.

In a still further advantageous embodiment of the invention, the detection unit for detecting foreign objects is a magnetic field transversing the path of the meat products. As most minced meat products are packaged in plastic or Styrofoam trays, these will not influence the magnetic field. However, any magnetic item passing the field will have an influence on the magnetic field, and thereby indicated that some foreign magnetic object has become lodged in the meat portion. The magnetic items which are of special concern relating to food items and especially minced meat products is pieces of machinery or especially the tips of butcher's knives or steel gloves which have broken off during the initial cutting of the meat carcasses. By incorporating such a detection unit into the quality control system it becomes possible to verify that products leaving the production facility for the consumer do not contain these types of foreign objects. Should a meat portion, however, contain a magnetic item, the data collection unit in the shape of the computing unit may be programmed to generate an appropriate response, for example an alarm or otherwise indicate that the particular meat portion needs to be rejected.

In a further advantageous embodiment of the invention the control system is provided with a device for rejection meat portions falling outside pre-specified parameters is arranged in conjunction with the conveying belt, where said device comprises means for registering portions with are rejected and transmitting the information to the computing unit, and further guiding said portions off the conveying belt.

As mentioned above it is important that meat products and especially minced meat is produced under controlled circumstances both with respect to health, hygiene and documentation of the products, but also such that the consumer will be able to be informed of the origin, correct processing, etc. of the product offered for sale. The provision of a computing unit for collecting data from all the sensors such as the light device, the detection unit for detecting foreign objects, the check weighing, etc. in cooperation with suitable software in the computing unit defining criteria for accepting or not accepting the collected data relating to the specific meat portion, makes it possible to assure that the quality of the product when leaving the production line is both verified, but also within the predetermined limits. If, however, one or more of the parameters are exceeded, the computing unit may transmit this to the rejection device such that the particular meat portion or a number of meat portions are rejected and thereby not transported on in the system, for example to the consumer, but are corrected, and optionally destroyed or treated according to the possibilities relating to the collected data. The rejection unit may be in the shape of an arm pushing the meat portion off the conveyor belt onto a second conveying belt which will facilitate storage of the rejected meat portions until further handling or in holding bins.

In a further advantageous embodiment, the control system is also provided with a labelling unit which is arranged in conjunction with the conveying belt where said unit receives information about the specific meat portion passing the labelling unit and attaches a label containing information about the specific meat portion to said portion, where the information may comprise one or more of the following or other data: date, time, batch number, weight of the portion, fat content, liquid content, type of meat, origin of meat, maximum and minimum temperature through meat processing, data identifying the processing plant. In this manner, it becomes possible to provide all the important information collected during the production process onto the specific product itself. Returning to the question of fat content, it is customary to produce different selections, i.e. one batch of minced meat containing 6-8% fat, another batch 8-10% fat, etc. As explained above, these limits and the traditional way of determining these limits is based on assumptions and experience, and may therefore often prove to be outside the limits specified on the product itself. However, by using the quality control system of the present invention in conjunction with a labelling unit, it is possible to provide specific information on the particular meat product in the package which the consumer is to buy. In this manner, the consumer is provided with exact information about the product.

In a further advantageous embodiment of the invention, the control system comprises a computing unit which:
a) collects data from one or more devices in the system;
b) optionally stores the data in a database, where data relating to a specific portion is compared to predefined parameters defining characteristics such as maximum/minimum fat content, weight of portion, liquid content interval, temperature intervals, time, date, type of meat;
c) where said predefined parameters defines criteria, for accept or reject of portion;
d) transmits relevant information to the labelling unit which in turn generates portion specific labels for the specific portion;
e) optionally a production quality report is generated containing data collected from the system, optionally further processed to include graphical presentation of the production data, generated averages and other relevant indicators;
f) optionally comprises a modem by wire or wireless for communicating with a remote applicant.

Depending on the environment in which the computing unit and the quality control system is to operate, it might be advantageous to keep the actual computing unit in a remote position such as for example a control room, or in other circumstances maintain the computing unit as an integral part of the system. Furthermore, the provision of a modem makes it possible to carry out maintenance and service, system upgrades, etc. from a remote location.

The collection of data from the quality control system into storage means such as for example a database, provides the possibility of generating reports on the performance of the entire meat production facility over time, such that tendencies or abnormalities may be detected and corrective measures initiated. The system may also be programmed to take out random samples. The samples may then be investigated using laboratory techniques, or other methods in order to verify that the system is collecting correct and reliable data. In this manner a reliable quality check system is provided.

Also, the collected data in a further advantageous embodiment may be compared to intervals outside which intervals the collected data initiates an alarm routine, optionally comprising a shut-down of the installation, audible alarms, generating of e-mail and/or SMS warning communications or other means of alerting to the alarm situation. In addition to being able to collect and register process parameters during the meat producing facility, and especially at the quality control system, it is also important to be able to compare and verify that the production of the meat portions are within the specified limits, and in case that these are exceeded generate a signal which will alert appropriate personal or generate corrective measures. This may be done as mentioned above by generating signals which according to the installation may reach appropriate personal as quickly as possible.

### Brief description of the drawings

The invention will now be explained in more detail with reference to the accompanying drawing. It should, however, be mentioned that the detailed description only describes principal embodiments of the invention, whereas other embodiments may be contemplated within the scope of the appended claims.
- Fig. 1: illustrates a schematic construction of a quality control system according to one embodiment of the invention,
- fig. 2: illustrates a further, more advanced embodiment of the invention.

### Detailed description of the preferred embodiment

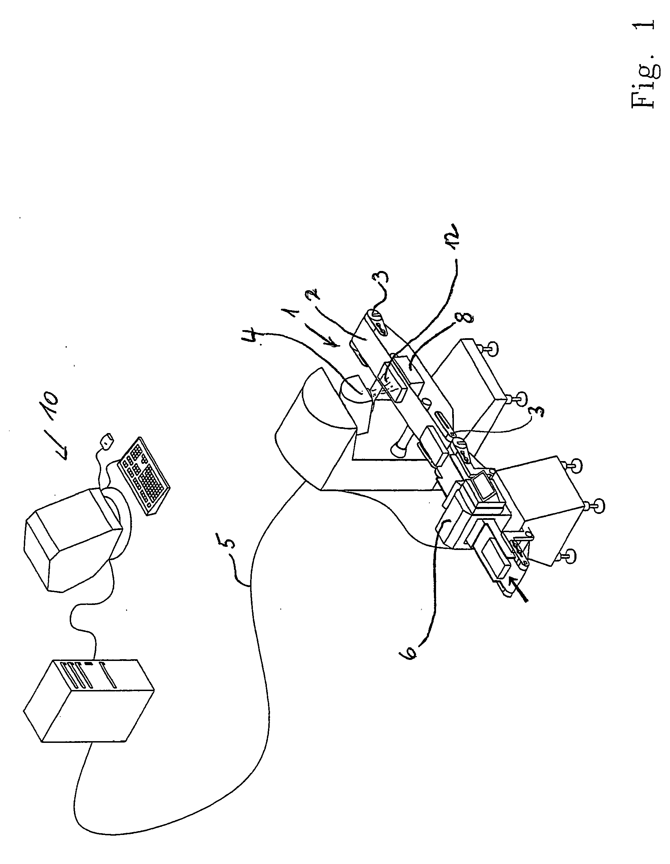

In fig. 1 is illustrated a schematic overview of a quality control system according to one embodiment of the invention. The system comprises a conveying belt 1 in the shape of an endless belt 2 which rotates about two rollers 3 arranged at either end of the conveying belt in a conventional manner. Above the belt 1 and arranged to direct a light 12 towards products placed on the belt 1 a non-contact light device 4 is arranged. The light device comprises a source of light, for example halogen, which will be directed towards the belt 1 through a lens, and optionally a filter unit, such that the light has a very well-defined wave length spectrum. In the non-contacting means 4, one or more sensors are arranged which will detect the reflected and absorbed wavelength in the products passing the light beam. In this manner, it is possible to collect data in the shape of absorbed and reflected wavelengths about the product placed below. The non-contact means, i.e. the light device, is in communication, in the illustration by wire 5, with the computing unit 10. The communication between the devices may be carried out by wireless means or by physical wire as indicated in fig. 1.

Also arranged in connection with the conveying belt 1 is a detection unit 6, which detection unit is provided in order to generate a magnetic field 7 such that magnetic objects passing through the magnetic field 7 will generate a signal in the detection unit 6 which may be transmitted to the computing unit 10. The magnetic objects which may be present in the products passing the magnetic field are typically tips of butcher knives, pieces of machinery, or other small metal objects deriving from the meat production facility.

Foreign objects may also be detected by the non-contact light means in that, if pieces of bone, cartilage, plastics or other foreign non-magnetic objects are present in the surface layers of the meat products passing underneath the light beam, these will reflect/absorb light at different wavelengths as compared to meat and/or fat, which the one or more sensors in the non-contact means 4 will be able to detect and to transmit to the computing unit 10. The software provided in the computing unit must, accordingly, be programmed to register and recognize these wavelengths patterns and identify them as foreign objects such as for example bone, cartilage, plastics or the like.

The control system further comprises a check weighing arrangement 8, which in this embodiment is illustrated as being build into the conveyor structure.

Alternatively, the check weighing may be conducted by a separate weighing unit arranged adjacent and independent to the conveying belt 1, depending on the physical constitution of the quality control system.

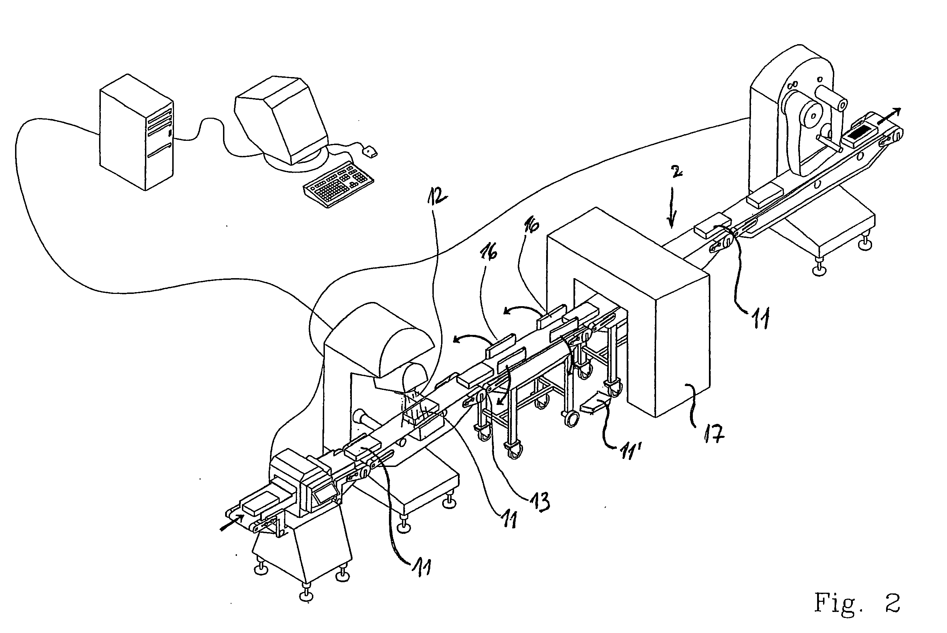

In fig. 2 is illustrated another embodiment of the invention comprising all the features of the system as described with reference to fig. 1, but additionally devices for rejecting and labelling meat portions have been added. Reference numbers depict the same features throughout the application.

In fig. 2 a number of trays 11 are being transported on the conveying belt 2. The trays contain finished minced meat portions which are to be transported on to the consumer. The trays 11 travel through the light beam 12 for non-contact control of the quality of the minced meat in the trays 11. Thereafter, the trays pass the magnetic field 7 in the device 6 for possible detection of magnetic foreign objects in the minced meat. Thereafter, the trays are transferred, for example by means of a bridge 13, to a device 6 for rejecting trays 11 which do not fulfil the requirements which have been preprogrammed in the software contained in the computing unit 10. In fig. 2 a tray 11' is depicted as having been rejected for failing to fulfil the predefined criteria, and is therefore taken out of the line of trays 11, which otherwise are ready to be forwarded to the consumer. Depending on the production facility and possibly the reason for rejecting the tray 11', the rejected minced meat portion may be reused or used for other purposes.

After the trays have passed the device 16 for rejecting/accepting the trays 11, the trays are conveyed on to a labelling unit 14. A packaging unit 17 may be provided before the labelling unit 14. The packaging unit will apply for example a thin plastic foil around each tray 11.

The labelling unit 14 will, depending on the input from the other devices in the production line and the quality control system, be programmed by the computing unit to generate a label which is specific for the particular tray. Depending on the desire of the customer, any information collected during the production process may be printed on the label which is attached to the minced meat portion in the labelling unit. In the art, a number of labelling systems are known, which both will be able to attach a label to a foil wrapped around the minced meat portion or a direct labelling on the meat items themselves.

Consequently, in this embodiment it is necessary that the device for rejecting/accepting the trays 11 as well as the labelling unit 14 are in communication with the computing unit 10. As is true for all other devices in the quality control system, the communication means may be in the shape of wires or may be in the shape of wireless means.

The computing unit may further be equipped with an alarm generating facility, such that the software detecting meat portions passing in the trays through the quality control system which has parameters outside the pre-specified intervals generate an alarm signal which may be transmitted to responsible personnel by for example wireless means, as SMS messages or emails. The computing unit may also be programmed to generate automatic responses in response to the incoming data. The invention has now been schematically described with respect to the two figures 1 and 2, but these examples should not be construed as being limiting on the scope of the invention, as the scope of the invention is defined by the appended claims.

## Claims

1. Quality control system for continuous control of meat products where the system comprises a conveyor (2) onto which the meat products (11) are placed and conveyed, where non-contact means (4) for registering the fat and/or protein and/or moisture content of the meat products is provided for continuous registration as the meat products passes the non-contact means on the conveyor, and further a check weighing system (8) comprising a detection unit (6) for detecting foreign objects arranged in connection with the conveyor, and that input from the non-contact fat and/or protein and/or moisture registering means, the weighing system and the metal detection unit are collected in a data storage means wherein a labelling unit (14) is arranged in conjunction with the conveying belt where said unit receives information about a specific meat portion passing the labelling unit and attaches a label containing information retrieved from the data storage means about the specific meat portion to said portion, where the information comprises one or more of the following data: fat content, moisture content, protein content.

2. Quality control system according to claim 1, wherein the meat products are minced meat in pre-specified weight portions and that the meat portions are packaged in trays.

3. Quality control system according to claim 1 or 2. wherein the non-contact means is a light device comprising a source of halogen light (4) and at least one sensor for receiving/monitoring and transmitting the reflected wavelengths from the products treated with the light to a computing unit, and further that the light device may comprise a non-contact temperature registration means.

4. Quality control system according to claim 1 or 2, wherein the check weighing system is integral with the conveyor belt, and that the check weighing is in communication with a computing unit (10).

5. Quality control system according to any preceding claim, wherein the detection unit for detecting foreign objects is a magnetic field transversing the path of the meat products.

6. Quality control system according to any preceding claim, wherein a device (6) for rejecting meat portions falling outside pre-specified parameters is arranged in conjunction with the conveying belt, where said device comprises means for registering portions with are rejected and transmitting the information to the computing unit, and further guiding said portions off the conveying belt.

7. Quality control system according to any preceding claim, wherein the computing unit:
g) collects data from one or more devices in the system;
h) optionally stores the data in a database, where data relating to a specific portion is compared to predefined parameters defining characteristics such as maximum/minimum fat content, weight of portion, liquid content interval, temperature intervals, time, date, type of meat;
i) where said predefined parameters defines criteria for accept or reject of portion;
j) transmits relevant information to the labelling unit which in turn generates portion specific labels for the specific portion;
k) optionally a production quality report is generated containing data collected from the system, optionally further processed to include graphical presentation of the production data, generated averages and other relevant indicators;
l) optionally comprises a modem by wire or wireless for communicating with a remote applicant.

8. Quality control system according to claim 7, wherein the collected data is further compared to intervals outside which intervals the collected data initiates an alarm routine, optionally comprising a shut-down of the installation, audible alarms, generating of e-mail and/or SMS warning communications or other means of alerting to the alarm situation.

## Patentansprüche

1. Qualitätsüberwachungssystem zur ständigen Überwachung von Fleischprodukten, wobei das System eine Fördereinrichtung (2) aufweist, auf dem die Fleischprodukte (11) angebracht und gefördert werden, wobei nicht-berührende Mittel (4) zum Nachweisen des Fett- und/oder Protein- und/oder Feuchtegehaltes der Fleischprodukte zum ständigen Nachweisen vorgesehen sind, wenn die Fleischprodukte auf der Fördereinrichtung an den nicht-berührenden Mitteln vorbeigeführt werden, und weiterhin ein in Verbindung mit der Fördereinrichtung angeordnetes, eine Ermittlungsvorrichtung (6) zur Ermittlung von Fremdobjekten aufweisendes Kontrollwaagesystem (8) aufweist, und dass Eingaben aus dem nicht-berührenden Fett- und/oder Protein- und/oder Feuchtenachweismittel, dem Waagesystem und der Metall-Ermittlullgseinheit in einem Datenspeicher gesammelt werden, wobei die Etikettiereinheit (14) in Verbindung mit dem Förderband angeordnet ist, wobei Auskünfte über eine spezifische, an der Etikettiereinheit vorbeilaufende Fleischmenge der erwähnten Einheit zugeführt werden und die Einheit ein mit vom Dataspeicher erhaltenen Auskünften über die spezifische Fleischmenge versehenes Etiket auf der Fleischmenge befestigt, wobei die Auskünfte eine oder mehrere der folgenden Angaben aufweist: Fettgehalt, Feuchtegehalt, Proteingehalt.

2. Qualitätsüberwachungssystem nach Anspruch 1, wobei die Fleischprodukte zerkleinertes Fleisch in voraus festgelegten Gewichtmengen sind, und daß die Fleischmengen in Tabletten verpackt sind.

3. Qualitätsüberwachungssystem nach Ansprüchen 1 oder 2, wobei das nicht-berührende Mittel eine Lichteinrichtung ist, die eine Halogenlichtquelle (4) und wenigstens einen Meßwertgeber zum Empfangen/Überwachen und Übermitteln an einen Rechner von den von den lichtbehandelten Fleischmengen reflektierten Wellenlängen aufweist, und daß die Lichteinrichtung weiter ein berührungsfreies Temperaturnachweismittel aufweisen kann.

4. Qualitätsüberwachungssystem nach Anspruch 1 oder 2, wobei das Kontrollwaagesystem mit dem Förderband integriert ist, und daß das Kontrollwiegen mit einer Rechnereinheit (10) verbunden ist.

5. Qualitätsüberwachungssystem nach irgendeinem der vorhergehenden Ansprüche, wobei die Ermittlungsvorrichtung zur Ermittlung von Fremdobjekten ein den Förderweg der Fleischprodukte kreuzendes Magnetfeld ist.

6. Qualitätsüberwachungssystem nach irgendeinem der vorhergehenden Ansprüche, wobei eine Vorrichtung (6) zum Ausscheiden der von den im voraus festgelegten Parametern abweichenden Fleischmengen vorgesehen und mit dem Förderband verbunden ist, wobei die Vorrichtung Mittel zum Nachweisen von ausgeschiedenen Mengen aufweist, die Auskünfte dem Rechner übermittelt und die Mengen vom Förderband weglenkt.

7. Qualitätsüberwachungssystem nach irgendeinem der vorhergehenden Ansprüche, wobei der Rechner:
g) Daten von einer oder mehreren Vorrichtungen im System einsammelt;
h) auf Wunsch die Daten in einem Datenbank speichert, wobei die eine spezifische Menge betreffenden Daten mit vordefinierten Parametern verglichen werden, welche Parameter die Kennzeichen, sowie maximalen/minimalen Fettgehalt, Mengengewicht, Flüssigkeitsgehaltsintervall, Temperaturintervalle, Zeit, Datum, Fleischtype definieren;
i) wo die vordefinierten Parameter Kriterien für Annahme oder Ausscheiden der Menge definieren;
j) die relevanten Auskünfte zur Etikettiereinheit weiterleitet, die wiederum mengenspezifische Etiketten für die spezifische Menge erzeugt;
k) wo auf Wunsch ein Herstellungsqualitätsbericht mit vom System geholten Daten erzeugt und auf Wunsch zur Anzeige von graphischer Darstellung von Produktionsdaten, erzeugten Durchschnitten und anderen relevanten Angaben weiterbehandelt wird;
l) auf Wunsch ein drahtversehenes oder drahtloses Modem zur Verbindung mit einem Fernbenutzer aufweist.

8. Qualitätsüberwachungssystem nach Anspruch 7, wobei weiter die eingesammelten Daten mit Intervallen verglichen werden, wobei die eingesammelten Daten außerhalb dieser Intervalle eine Alarmroutine einleitet, die auf Wunsch ein Abschalten der Anlage, hörbare Alarme, Erzeugung eines E-Mail- und/oder SMS-Warnungsbescheides oder anderer Warnungen vor dem Alarmzustand umfaßt.

## Revendications

1. Système de contrôle de qualité pour le contrôle continu de produits à base de viande, où le système comprend un transporteur (2) sur lequel les produits à base de viande (11) sont placés et transportés, où des moyens sans contact (4) pour enregistrer la teneur en graisse et/ou en protéines et/ou en humidité des produits à base de viande sont prévus pour un enregistrement continu alors que les produits à base de viande passent devant les moyens sans contact sur le transporteur, et en outre un système de pesage de contrôle (8) comprenant une unité de détection (6) pour détecter des objets étrangers agencés en relation avec le transporteur, et dans lequel les entrées des moyens sans contact d'enregistrement de teneur en graisse et/ou en protéines et/ou en humidité, du système de pesage et de l'unité de détection de métal sont collectées dans des moyens de mémorisation de données, dans lequel une unité d'étiquetage (14) est agencée conjointement avec la courroie de transport, où ladite unité reçoit des informations concernant une portion de viande spécifique passant devant l'unité d'étiquetage et fixe une étiquette contenant des informations récupérées auprès des moyens de mémorisation de données concernant la portion de viande spécifique pour ladite portion, où les informations comprennent une ou plusieurs des données suivantes : une teneur en graisse, une teneur en humidité, une teneur en protéines.

2. Système de contrôle de qualité selon la revendication 1, dans lequel les produits à base de viande sont de la viande hachée en des portions de poids spécifié au préalable et les portions de viande sont conditionnées dans des plateaux.

3. Système de contrôle de qualité selon la revendication 1 ou 2, dans lequel les moyens sans contact sont un dispositif lumineux comprenant une source de lumière halogène (4) et au moins un capteur pour recevoir/surveiller et transmettre les longueurs d'onde réfléchies par les produits traités par la lumière à une unité de calcul, et dans lequel, en outre, le dispositif lumineux peut comprendre des moyens d'enregistrement de température sans contact.

4. Système de contrôle de qualité selon la revendication 1 ou 2, dans lequel le système de pesage de contrôle est d'un seul tenant avec la courroie de transport, et dans lequel le pesage de contrôle est en communication avec une unité de calcul (10).

5. Système de contrôle de qualité selon l'une quelconque des revendications précédentes, dans lequel l'unité de détection pour détecter des objets étrangers est un champ magnétique traversant le trajet des produits à base de viande.

6. Système de contrôle de qualité selon l'une quelconque des revendications précédentes, dans lequel un dispositif (6) pour rejeter les portions de viande tombant en-dehors de paramètres spécifiés au préalable est agencé conjointement avec la courroie de transport, où ledit dispositif comprend des moyens pour enregistrer les portions qui sont rejetées et transmettre les informations à l'unité de calcul, et en outre guider lesdites portions hors de la courroie de transport.

7. Système de contrôle de qualité selon l'une quelconque des revendications précédentes, dans lequel l'unité de calcul :
g) collecte des données auprès d'un ou de plusieurs dispositifs dans le système ;
h) mémorise, optionnellement, les données dans une base de données, où les données concernant une portion spécifique sont comparées à des paramètres prédéfinis définissant des caractéristiques telles qu'une teneur en graisse maximum/minimum, le poids de la portion, l'intervalle de teneur en liquide, les intervalles de température, l'heure, la date, le type de viande ;
i) où lesdits paramètres prédéfinis définissent des critères d'acceptation ou de rejet de la portion ;
j) transmet des informations pertinentes à l'unité d'étiquetage qui, à son tour, génère des étiquettes spécifiques à une portion pour la portion spécifique ;
k) optionnellement, un rapport de qualité de production est généré contenant des données collectées auprès du système, optionnellement traitées davantage pour inclure une présentation graphique des données de production, des moyennes générées et d'autres indicateurs pertinents ;
l) comprend optionnellement un modem câblé ou sans fil pour communiquer avec un demandeur à distance.

8. Système de contrôle de qualité selon la revendication 7, dans lequel les données collectées sont en outre comparées à des intervalles, en-dehors desquels intervalles les données collectées lancent un sous-programme d'alarme, comprenant optionnellement un arrêt de l'installation, des alarmes audibles, la génération de communications d'avertissement par courrier électronique et/ou SMS ou d'autres moyens alertant de la situation d'alarme.
